# EUROPEAN PATENT SPECIFICATION

(11) **EP 4 404 905 B1**
(45) Date of publication and mention of the grant of the patent: **06.05.2026**
(21) Application number: 21957858.0
(22) Date of filing: 24.09.2021
(51) Int. Cl.: A61K 8/11, A61K 8/85, A61Q 5/02, A61K 8/02, A61Q 19/10, A61K 8/29

(54) **COMPOSITE OPACIFIER DISPERSION**
ZUSAMMENGESETZTE TRÜBUNGSMITTELDISPERSION
DISPERSION D'AGENT OPACIFIANT COMPOSITE

(43) Date of publication of application: 31.07.2024
(73) Proprietor: Dow Global Technologies LLC, Midland, MI 48674 (US); Dow Silicones Corporation, Midland, MI 48686-0994 (US)
(72) Inventor: WANG, Tao, Shanghai 201203 (CN); BAO, Xinyan, Shanghai 201203 (CN); GUO, Yunlong, Shanghai 201803 (CN); CHEN, Hongyu, Shanghai 201203 (CN); PANG, Xiaoyi, Shanghai 201102 (CN)
(74) Representative: Jewell, Catherine Mary
(86) International application number: PCT/CN2021/120210
(87) International publication number: WO 2023/044724

(56) References cited:
- EP-A1- 2 990 445
- WO-A1-2013/105556
- CN-A- 1 852 763
- CN-A- 101 365 412
- CN-A- 105 407 718
- CN-A- 109 966 174
- US-A1- 2019 053 991
- ROBERT Y LOCHHEAD ET AL: "A Review of Recent Advances in the Polymeric Delivery of Attributes in Cosmetics and Personal Care Products Precise Molecular Tailoring for Simultaneous Enablement of Contrasting Qualities", 1 January 2010 (2010-01-01), XP055586489, Retrieved from the Internet <URL:https://pubs.acs.org/doi/pdf/10.1021/bk-2010-1053.ch001> [retrieved on 20190507]

## Description

The present invention relates to a composite opacifier dispersion, comprising: a dispersion medium; a processing surfactant; and a plurality of composite opacifier particles, wherein the composite opacifier particles comprise metal oxide particles that are partially or completely encapsulated by a polycaprolactone polymer; wherein the metal oxide particles are selected from the group consisting of zinc oxide, titanium oxide and mixtures thereof; wherein the metal oxide particles have a z average particle size of > 100 nm as measured by dynamic light scattering; and wherein the composite opacifier particles have a z average particle size of > 150 nm to 2,500 nm as measured by dynamic light scattering.

In addition to cleaning performance, the aesthetic look and feel of a detergent is an important consideration for consumers. Thus, detergents typically contain a variety of ingredients that impact functionality, aesthetics, or both, including, for instance, surfactants, solvents, optional builder, and opacifiers.

Opacifiers are materials that make a liquid system opaque. Thus, opacifiers are used to modify the appearance or aesthetics of detergents, for instance, by transforming the liquid from clear or translucent to opaque. Opacifiers can provide a uniform, luxurious, "lotionized" appearance to a liquid product. Opacifiers are usually formed of submicron sized particles that are delivered to a formulation as a suspension of the particles in a solvent (typically water).

Since opacifiers are targeted to a formulation's aesthetics, it is generally desirable that their inclusion not interfere with the function of the formulation or otherwise negatively impact the formulation. For instance, opacifiers that exhibit limited compatibility with other materials in the formulation, have issues with stability, exhibit spotting or residue formation, are not favored. In addition, opacifiers that introduce large amounts of water into a formulation, e.g., by being effective only when used in large quantities, are also not favored, particularly for those formulations where limiting the quantity of water is desired, such as in concentrated detergents or unit dose packets.

Accordingly, there remains a need for more sustainable opacifiers for use in aqueous personal care rinse off compositions; particularly wherein the sustainable opacifiers perform on par with conventional styrene acrylic copolymer based opacifiers.

The present invention provides a composite opacifier dispersion, comprising: a dispersion medium; a processing surfactant; and a plurality of composite opacifier particles, wherein the composite opacifier particles comprise metal oxide particles that are partially or completely encapsulated by a polycaprolactone polymer; wherein the metal oxide particles are selected from the group consisting of zinc oxide, titanium oxide and mixtures thereof; wherein the metal oxide particles have a z average particle size of > 100 nm as measured by dynamic light scattering; and wherein the composite opacifier particles have a z average particle size of > 150 nm to 2,500 nm as measured by dynamic light scattering.

The present invention provides an aqueous personal care rinse off composition, comprising: a dermatologically acceptable aqueous vehicle; a dermatologically acceptable cleaning surfactant; and a composite opacifier dispersion, comprising: a dispersion medium; a processing surfactant; and a plurality of composite opacifier particles, wherein the composite opacifier particles comprise metal oxide particles that are partially or completely encapsulated by a polycaprolactone polymer; wherein the metal oxide particles are selected from the group consisting of zinc oxide, titanium oxide and mixtures thereof; wherein the metal oxide particles have a z average particle size of > 100 nm as measured by dynamic light scattering; and wherein the composite opacifier particles have a z average particle size of > 150 nm to 2,500 nm as measured by dynamic light scattering.

The present invention provides a method of cleaning at least one of mammalian skin and hair, comprising: (a) applying an aqueous personal care rinse off formulation according to the present invention to the skin or hair of a mammal; and (b) rinsing the aqueous personal care rinse off formulation from the skin or hair with a rinse water.

### DETAILED DESCRIPTION

We have surprisingly found that a composite opacifier dispersion comprising metal oxide particles partially or completely encapsulated by a polycaprolactone polymer can be stably incorporated into aqueous personal care rinse off compositions to provide opacification on par with conventional styrene acrylate copolymer based opacifiers, while increasing the overall sustainability of the aqueous personal care rinse off composition.

Unless otherwise indicated, ratios, percentages, parts, and the like are by weight.

As used herein, unless otherwise indicated, the phrase "molecular weight" or M_{W} refers to the weight average molecular weight as measured in a conventional manner with gel permeation chromatography (GPC) and poly(ethylene oxide) standards. GPC techniques are discussed in detail in Modern Size Exclusion Chromatography, W. W. Yau, J. J. Kirkland, D. D. Bly; Wiley-Interscience, 1979, and in A Guide to Materials Characterization and Chemical Analysis, J. P. Sibilia; VCH, 1988, p.81-84. Molecular weights are reported herein in units of Daltons, or equivalently, g/mol.

The term "dermatologically acceptable" as used herein and in the appended refers to ingredients that are typically used for topical application to the skin, and is intended to underscore that materials that are toxic when present in the amounts typically found in skin care compositions are not contemplated as part of the present invention.

Preferably, the composite opacifier dispersion of the present invention, comprises: a dispersion medium (preferably, 25 to 90 wt% (more preferably, 40 to 85 wt%; still more preferably, 50 to 80 wt%; most preferably, 60 to 75 wt%), based on weight of the composite opacifier dispersion, of the dispersion medium); a processing surfactant (preferably, 0.1 to 15 wt% (more preferably, 0.4 to 9 wt%; still more preferably, 0.6 to 5 wt%; most preferably, 1.25 to 4 wt%, based on weight of the composite opacifier dispersion, of the processing surfactant); and a plurality of composite opacifier particles (preferably 9.9 to 60 wt% (more preferably, 14.6 to 59.6 wt%; still more preferably, 19.4 to 49.4 wt%; most preferably, 23.75 to 36 wt%, based on weight of the composite opacifier dispersion, of the plurality of composite opacifier particles), wherein the composite opacifier particles comprise metal oxide particles that are partially or completely encapsulated by a polycaprolactone polymer; wherein the metal oxide particles are selected from the group consisting of zinc oxide, titanium oxide and mixtures thereof; wherein the metal oxide particles have a z average particle size of > 100 nm as measured by dynamic light scattering; and wherein the composite opacifier particles have a z average particle size of > 150 nm to 2,500 nm (preferably, 200 nm to 2,000 nm; more preferably, 500 nm to 1,800 nm; most preferably, 750 nm to 1,600 nm) as measured by dynamic light scattering (e.g., with a Beckman Coulter Particle Size Analyzer with a Universal Liquid Module).

Preferably, the composite opacifier dispersion of the present invention, comprises: 25 to 90 wt% (preferably, 40 to 85 wt%; more preferably, 50 to 80 wt%; most preferably, 60 to 75 wt%), based on weight of the composite opacifier dispersion, of a dispersion medium of a dispersion medium. More preferably, the composite opacifier dispersion of the present invention, comprises: 25 to 90 wt% (preferably, 40 to 85 wt%; more preferably, 50 to 80 wt%; most preferably, 60 to 75 wt%), based on weight of the composite opacifier dispersion, of a dispersion medium; wherein the dispersion medium comprises water. Most preferably, the composite opacifier dispersion of the present invention, comprises: 25 to 90 wt% (preferably, 40 to 85 wt%; more preferably, 50 to 80 wt%; most preferably, 60 to 75 wt%), based on weight of the composite opacifier dispersion, of a dispersion medium; wherein the dispersion medium is water (preferably, wherein the water is at least one of distilled and deionized).

Preferably, the composite opacifier dispersion of the present invention, comprises: 0.1 to 15 wt% (preferably, 0.4 to 9 wt%; more preferably, 0.6 to 5 wt%; most preferably, 1.25 to 4 wt%), based on weight of the composite opacifier dispersion, of a processing surfactant. More preferably, the composite opacifier dispersion of the present invention, comprises: 0.1 to 15 wt% (preferably, 0.4 to 9 wt%; more preferably, 0.6 to 5 wt%; most preferably, 1.25 to 4 wt%), based on weight of the composite opacifier dispersion, of a processing surfactant; wherein the processing surfactant is selected from the group consisting of an ionic surfactant, a nonionic surfactant and a cationic surfactant. Most preferably, the composite opacifier dispersion of the present invention, comprises: 0.1 to 15 wt% (preferably, 0.4 to 9 wt%; more preferably, 0.6 to 5 wt%; most preferably, 1.25 to 4 wt%), based on weight of the composite opacifier dispersion, of a processing surfactant; wherein the processing surfactant is a nonionic surfactant (preferably, wherein the nonionic surfactant has a weight average molecular weight of 5,000 to 150,000 Daltons (preferably, 5,000 to 100,000 Daltons; more preferably, 7,500 to 50,000 Daltons; most preferably, 10,000 to 25,000 Daltons).

Preferably, the composite opacifier dispersion of the present invention, comprises: 0.1 to 15 wt% (preferably, 0.4 to 9 wt%; more preferably, 0.6 to 5 wt%; most preferably, 1.25 to 4 wt%), based on weight of the composite opacifier dispersion, of a processing surfactant; wherein the processing surfactant is a nonionic surfactant selected from the group consisting of polyoxyalkylene surfactants, polyalkylene glycol esters, polyoxyethylene derivatives of fatty acid esters of polyhydric alcohols, fatty acid esters of polyalkoxylated polyhydric alcohols, polyalkoxylated natural fats and oils, polyalkylene oxide block copolymers, alkyl polyglucosides, sucrose esters and mixtures thereof (preferably, wherein the nonionic surfactant has a weight average molecular weight of 5,000 to 150,000 Daltons (preferably, 5,000 to 100,000 Daltons; more preferably, 7,500 to 50,000 Daltons; most preferably, 10,000 to 25,000 Daltons). More preferably, the composite opacifier dispersion of the present invention, comprises: 0.1 to 15 wt% (preferably, 0.4 to 9 wt%; more preferably, 0.6 to 5 wt%; most preferably, 1.25 to 4 wt%), based on weight of the composite opacifier dispersion, of a processing surfactant; wherein the processing surfactant is a poly(alkylene oxide) polymer (preferably, wherein the poly(alkylene oxide) polymer has a weight average molecular weight of 5,000 to 150,000 Daltons (preferably, 5,000 to 100,000 Daltons; more preferably, 7,500 to 50,000 Daltons; most preferably, 10,000 to 25,000 Daltons). Still more preferably, the composite opacifier dispersion of the present invention, comprises: 0.1 to 15 wt% (preferably, 0.4 to 9 wt%; more preferably, 0.6 to 5 wt%; most preferably, 1.25 to 4 wt%), based on weight of the composite opacifier dispersion, of a processing surfactant; wherein the processing surfactant is a poly(alkylene oxide) copolymer comprising residues of ethylene oxide (EO) and propylene oxide (PO) (preferably, wherein the nonionic surfactant has a weight average molecular weight of 5,000 to 150,000 Daltons (preferably, 5,000 to 100,000 Daltons; more preferably, 7,500 to 50,000 Daltons; most preferably, 10,000 to 25,000 Daltons). Most preferably, the composite opacifier dispersion of the present invention, comprises: 0.1 to 15 wt% (preferably, 0.4 to 9 wt%; more preferably, 0.6 to 5 wt%; most preferably, 1.25 to 4 wt%), based on weight of the composite opacifier dispersion, of a processing surfactant; wherein the processing surfactant is an ABA type triblock copolymer of ethylene oxide (EO) (A block) and propylene oxide (PO) (B block) (preferably, wherein the ABA type triblock copolymer has a weight average molecular weight of 5,000 to 150,000 Daltons (preferably, 5,000 to 100,000 Daltons; more preferably, 7,500 to 50,000 Daltons; most preferably, 10,000 to 25,000 Daltons).

Preferably, the composite opacifier dispersion of the present invention, comprises: 9.9 to 60 wt% (preferably, 14.6 to 59.6 wt%; more preferably, 19.4 to 49.4 wt%; most preferably, 23.75 to 36 wt%), based on weight of the composite opacifier dispersion, of a plurality of composite opacifier particles. More preferably, the composite opacifier dispersion of the present invention, comprises: 9.9 to 60 wt% (preferably, 14.6 to 59.6 wt%; more preferably, 19.4 to 49.4 wt%; most preferably, 23.75 to 36 wt%), based on weight of the composite opacifier dispersion, of a plurality of composite opacifier particles; wherein the composite opacifier particles comprise metal oxide particles that are partially or completely encapsulated by a polycaprolactone polymer; wherein the composite opacifier particles comprise metal oxide particles that are partially or completely encapsulated by a polycaprolactone polymer; wherein the metal oxide particles are selected from the group consisting of zinc oxide, titanium oxide and mixtures thereof; wherein the metal oxide particles have a z average particle size of > 100 nm as measured by dynamic light scattering; and wherein the composite opacifier particles have a z average particle size of > 150 nm to 2,500 nm (preferably, 200 nm to 2,000 nm; more preferably, 500 nm to 1,800 nm; most preferably, 750 nm to 1,600 nm) as measured by dynamic light scattering (e.g., with a Beckman Coulter Particle Size Analyzer with a Universal Liquid Module).

Preferably, the metal oxide particles are selected from the group consisting of zinc oxide, titanium oxide and mixtures thereof; wherein the metal oxide particles have a z average particle size of > 100 nm (preferably, 110 nm to 500 nm; more preferably, 150 nm to 400 nm; most preferably, 175 to 275 nm) as measured by dynamic light scattering (e.g, with a Brookhaven particle size analyzer). More preferably, the metal oxide particles include titanium dioxide particles having a z average particle size of > 100 nm (preferably, 110 nm to 500 nm; more preferably, 150 nm to 400 nm; most preferably, 175 to 275 nm) as measured by dynamic light scattering (e.g, with a Brookhaven particle size analyzer). Most preferably, the metal oxide particles are titanium dioxide particles having a z average particle size of > 100 nm (preferably, 110 nm to 500 nm; more preferably, 150 nm to 400 nm; most preferably, 175 to 275 nm) as measured by dynamic light scattering (e.g, with a Brookhaven particle size analyzer).

Preferably, the metal oxide particles have a hydrophobic surface treatment (e.g., a polysiloxane surface coating).

Preferably, the polycaprolactam polymer has a weight average molecular weight of 10,000 to 1,000,000 Daltons (preferably, 25,000 to 250,000 Daltons; more preferably, 50,000 to 150,000 Daltons; most preferably, 75,000 to 125,000 Daltons).

Preferably, the aqueous personal care rinse off composition of the present invention is selected from the group consisting of a shampoo formulation, a conditioning shampoo formulation, a body wash formulation, an exfoliating body wash formulation, a facial wash formulation, an exfoliating facial wash formulation, a liquid hand soap formulation, a sulfate-free cleansing formulation and a mild cleansing formulation. More preferably, the aqueous personal care rinse off composition of the present invention is selected from the group consisting of a body wash formulation, a shampoo formulation and a conditioning shampoo formulation. Most preferably, the aqueous personal care rinse off composition of the present invention is a shampoo formulation.

Preferably, the aqueous personal care rinse off composition of the present invention, comprises: a dermatologically acceptable aqueous vehicle (preferably, wherein the aqueous personal care rinse off composition comprises 25 to 99 wt% (preferably, 30 to 95 wt%; more preferably, 40 to 90 wt%; most preferably, 70 to 85 wt%), based on weight of the aqueous personal care rinse off composition, of the dermatologically acceptable aqueous vehicle); a dermatologically acceptable cleaning surfactant (preferably, wherein the aqueous personal care rinse off composition comprises 0.5 to 60 wt% (preferably, 1 to 50 wt%; more preferably, 5 to 30 wt%; most preferably, 7 to 20 wt%), based on weight of the aqueous personal care rinse off composition, of the dermatologically acceptable cleaning surfactant); and a plurality of composite opacifier particles (preferably, wherein the aqueous personal care rinse off composition comprises 0.05 to 10 wt% (preferably, 0.1 to 7.5 wt%; more preferably, 0.5 to 5 wt%; most preferably, 0.75 to 3.0 wt%), based on weight of the aqueous personal care rinse off composition, of the plurality of composite opacifier particles), wherein the composite opacifier particles comprise metal oxide particles that are partially or completely encapsulated by a polycaprolactone polymer; wherein the metal oxide particles are selected from the group consisting of zinc oxide, titanium oxide and mixtures thereof; wherein the metal oxide particles have a z average particle size of > 100 nm as measured by dynamic light scattering; and wherein the composite opacifier particles have a z average particle size of > 150 nm to 2,500 nm (preferably, 200 nm to 2,000 nm; more preferably, 500 nm to 1,800 nm; most preferably, 750 nm to 1,600 nm) as measured by dynamic light scattering (e.g., with a Beckman Coulter Particle Size Analyzer with a Universal Liquid Module).

Preferably, the aqueous personal care rinse off composition of the present invention, comprises: 25 to 99 wt% (preferably, 30 to 95 wt%; more preferably, 40 to 90 wt%; most preferably, 70 to 85 wt%), based on weight of the aqueous personal care rinse off composition, of a dermatologically acceptable aqueous vehicle. More preferably, the aqueous personal care rinse off composition of the present invention, comprises: 25 to 99 wt% (preferably, 30 to 95 wt%; more preferably, 40 to 90 wt%; most preferably, 70 to 85 wt%), based on weight of the aqueous personal care rinse off composition, of a dermatologically acceptable aqueous vehicle; wherein the dermatologically acceptable aqueous vehicle comprises water. Still more preferably, the aqueous personal care rinse off composition of the present invention, comprises: 25 to 99 wt% (preferably, 30 to 95 wt%; more preferably, 40 to 90 wt%; most preferably, 70 to 85 wt%), based on weight of the aqueous personal care rinse off composition, of a dermatologically acceptable aqueous vehicle; wherein the dermatologically acceptable aqueous vehicle is selected from the group consisting of water and an aqueous C₁₋₄ alcohol mixture. Most preferably, the aqueous personal care rinse off composition of the present invention, comprises: 25 to 99 wt% (preferably, 30 to 95 wt%; more preferably, 40 to 90 wt%; most preferably, 70 to 85 wt%), based on weight of the aqueous personal care rinse off composition, of a dermatologically acceptable aqueous vehicle, wherein the dermatologically acceptable aqueous vehicle is water.

Preferably, the water used in the aqueous personal care rinse off composition of the present invention is at least one of distilled water and deionized water. More preferably, the water used in the aqueous personal care rinse off composition of the present invention is distilled and deionized.

Preferably, the aqueous personal care rinse off composition of the present invention comprises 0.5 to 60 wt% (preferably, 1 to 50 wt%; more preferably, 5 to 30 wt%; most preferably, 7 to 20 wt%), based on weight of the aqueous personal care rinse off composition, of a dermatologically acceptable cleaning surfactant. More preferably, the aqueous personal care rinse off composition of the present invention comprises 0.5 to 60 wt% (preferably, 1 to 50 wt%; more preferably, 5 to 30 wt%; most preferably, 7 to 20 wt%), based on weight of the aqueous personal care rinse off composition, of a dermatologically acceptable cleaning surfactant; wherein the dermatologically acceptable cleaning surfactant is selected from the group consisting of alkyl polyglucosides (e.g., lauryl glucoside, coco-glucoside, decyl glucoside), glycinates (e.g., sodium cocoyl glycinate), betaines (e.g., alkyl betaines such as trimethylglycine and cetyl betaine; and amido betaines such as cocamidopropyl betaine), taurates (e.g., sodium methyl cocoyl taurate), glutamates (e.g., sodium cocoyl glutamate), sarcosinates (e.g., sodium lauroyl sarcosinate), isethionates (e.g., sodium cocoyl isethionate, sodium lauroyl methyl isethionate), sulfoacetates (e.g., sodium lauryl sulfoacetate), alaninates (e.g., sodium cocoyl alaninate), amphoacetates (e.g., sodium cocoamphoacetate), sulfates (e.g., sodium laureth sulfate (SLES)), sulfonates (e.g., sodium C₁₄₋₁₆ olefin sulfonate), succinates (e.g., disodium lauryl sulfosuccinate); fatty alkanolamides (e.g., cocamide monoethanolamine, cocamide, diethanolamine, soyamide diethanolamine, lauramide diethanolamine, oleamide monoisopropanolamine, stearamide monoethanolamine, myristamide monoethanolamine, lauramide monoethanolamine, capramide diethanolamine, ricinoleamide diethanolamine, myristamide diethanolamine, stearamide diethanolamine, oleylamide diethanolamine, tallowamide diethanolamine, lauramide monoisopropanolamine, tallowamide monoethanolamine, isostearamide diethanolamine, isostearamide diethanolamine, isostearamide monoethanolamine) and mixtures thereof. Still more preferably, the aqueous personal care rinse off composition of the present invention comprises 0.5 to 60 wt% (preferably, 1 to 50 wt%; more preferably, 5 to 30 wt%; most preferably, 7 to 20 wt%), based on weight of the aqueous personal care rinse off composition, of a dermatologically acceptable cleaning surfactant; wherein the dermatologically acceptable cleaning surfactant includes a mixture of sodium laureth sulfate (SLES) and cocamidopropyl betaine. Most preferably, the aqueous personal care rinse off composition of the present invention comprises 0.5 to 60 wt% (preferably. 1 to 50 wt%; more preferably, 5 to 30 wt%; most preferably, 7 to 20 wt%), based on weight of the aqueous personal care rinse off composition, of a dermatologically acceptable cleaning surfactant; wherein the dermatologically acceptable cleaning surfactant is a mixture of sodium laureth sulfate (SLES) and cocamidopropyl betaine.

Preferably, the aqueous personal care rinse off composition of the present invention, optionally, further comprises at least one additional ingredient selected from the group consisting of an antimicrobial agent; a rheology modifier; a soap; a colorant; a pH adjusting agent; an antioxidant (e.g., butylated hydroxytoluene); a humectant (e.g., glycerin, sorbitol, monoglycerides, lecithins, glycolipids, fatty alcohols, fatty acids, polysaccharides, sorbitan esters, polysorbates (e.g., Polysorbate 20, Polysorbate 40, Polysorbate 60, and Polysorbate 80), diols (e.g., propylene glycol), diol analogs, triols, triol analogs, cationic polymeric polyols); a foaming agent; an emulsifying agent; a fragrance; a chelating agent; a preservative (e.g., benzoic acid, sorbic acid, phenoxyethanol); a bleaching agent; a lubricating agent; a sensory modifier; a sunscreen additive; a vitamin; a protein/amino acid; a plant extract; a bioactive agent; an anti-aging agent; a penetrant; an anti-static agent; an absorbent; a hard particle; a soft particle; a slip agent; a pearlizing agent; a salt (e.g., a moisturizing salt) and mixtures thereof. More preferably, the skin cleansing formulation of the present invention, optionally, further comprises at least one additional ingredient selected from the group consisting of at least one of an antimicrobial agent, a rheology modifier, a colorant and a pH adjusting agent.

Preferably, the aqueous personal care rinse off composition of the present invention, further comprises a rheology modifier. More preferably, the aqueous personal care rinse off formulation of the present invention, further comprises a rheology modifier; wherein the rheology modifier is selected to increase the viscosity of the aqueous personal care rinse off composition (preferably without substantially modifying the other properties of the aqueous personal care rinse off composition). Still more preferably, the aqueous personal care rinse off composition of the present invention, further comprises 0 to 10 wt% (preferably, 0.05 to 5 wt%; more preferably, 0.1 to 2.5 wt%; most preferably, 0.15 to 2.0 wt%), based on weight of the aqueous personal care rinse off composition, of a rheology modifier. Yet more preferably, the aqueous personal care rinse off composition of the present invention, further comprises 0 to 10 wt% (preferably, 0.05 to 5 wt%; more preferably, 0.1 to 2.5 wt%; most preferably, 0.15 to 2.0 wt%), based on weight of the aqueous personal care rinse off composition, of a rheology modifier; wherein the rheology modifier is selected from the group consisting of sodium chloride, cellulose, a modified cellulose, xanthan gum, an acrylates copolymer and mixtures thereof. Most preferably, the aqueous personal care rinse off composition of the present invention, further comprises 0 to 10 wt% (preferably, 0.05 to 5 wt%; more preferably, 0.1 to 4 wt%; most preferably, 0.15 to 3.5 wt%), based on weight of the aqueous personal care rinse off composition, of a rheology modifier; wherein the rheology modifier includes a sodium chloride.

Preferably, the aqueous personal care rinse off composition of the present invention, further comprises an antimicrobial agent. More preferably, the aqueous personal care rinse off composition of the present invention, further comprises an antimicrobial agent; wherein the antimicrobial agent is selected from the group consisting of phenoxyethanol, benzoic acid, benzyl alcohol, sodium benzoate, DMDM hydantoin, 2-ethylhexyl glyceryl ether, isothiazolinone (e.g., methylchloroisothiazolinone, methylisothiazolinone) and mixtures thereof. Still more preferably, the aqueous personal care rinse off composition of the present invention, further comprises an antimicrobial agent; wherein the antimicrobial agent includes at least one of phenoxyethanol and 2-ethylhexyl glyceryl ether. Most preferably, the aqueous personal care rinse off composition of the present invention, further comprises an antimicrobial agent; wherein the antimicrobial agent is a mixture of phenoxyethanol and 2-ethylhexyl glyceryl ether.

Preferably, the aqueous personal care rinse off composition of the present invention, further comprises a pH adjusting agent. More preferably, the aqueous personal care rinse off composition of the present invention, further comprises a pH adjusting agent; wherein the aqueous personal care rinse off composition is a body wash formulation. Most preferably, the aqueous personal care rinse off composition of the present invention, further comprises a pH adjusting agent; wherein the aqueous personal care rinse off composition is a body wash formulation and wherein the body wash formulation has a pH of 4.5 to 9 (preferably, 5 to 8; most preferably, 6 to 7). Preferably, the pH adjusting agent is selected from the group consisting of at least one of citric acid, lactic acid, hydrochloric acid, aminoethyl propanediol, triethanolamine, monoethanolamine, sodium hydroxide, potassium hydroxide, amino-2-methyl-1-propanol.

Preferably, the aqueous personal care rinse off composition of the present invention, further comprises a colorant.

Preferably, the method of cleaning at least one of mammalian skin and hair of the present invention, comprises: applying an aqueous personal care rinse off composition of the present invention to the skin or hair of a mammal; and rinsing the aqueous personal care rinse off composition from the skin and/or hair with a rinse water. More preferably, the method of cleaning at least one of human skin and hair of the present invention, comprises: applying an aqueous personal care rinse off composition of the present invention to the skin or hair of a mammal; and rinsing the aqueous personal care rinse off composition from the skin and/or hair with a rinse water.

Some embodiments of the present invention will now be described in detail in the following **Examples.**

Reagents used in the **Examples** are described in **TABLE 1.**

**TABLE 1**

| **Identifier** | **Description** |
|---|---|
| CAPA^{™} 6500 | Polycaprolactone (PCL) weight average molecular weight 96,000 Daltons available from Perstop Co. Ltd. |
| CAPA^{™} 6800 | Polycaprolactone (PCL) weight average molecular weight 86,000 Daltons available from Perstop Co. Ltd. |
| Kronos R2233 | Titanium dioxide available from Kronos Worldide, Inc. |
| Pluronic F-180 | PPO-PEO deblock copolymer weight average molecular weight 14,600 Daltons available from BASF |
| ECOSURF^{™} EH-40 | Non-ionic alcohol ethoxylate surfactant available from The Dow Chemical Company |
| DOWFAX^{™} AS-801 | Alkyl alkoxylate sulfate surfactant available from The Dow Chemical Company |
| PVA (GH-17S) | Polyvinyl alcohol available from Nippon Synthetic Chemical Industry Co., Ltd. |
| SLES (70%) | Sodium laureth sulfate surfactant available from SCRC |
| CAPB | Cocamidopropyl betaine surfactant available from SCRC |
| Glycerine | Glycerine humectant available from SCRC |
| Euxyl^{®} PE 9010 | Phenoxyethanol (and) ethylhexylglycerin available from Schulke & Mayr |
| NaCl | Sodium chloride |

### Synthesis S1: Polycaprolactone/TiO₂ composite

In **Synthesis S1**, polycaprolactone (CAPA^{™} 6500) and TiO₂ (Kronos R2233) were pre-compounded in a Leistritz ZSE27 MAXX twin screw extruder. The polycaprolactone pellets were fed into the extruder at a rate of 12 kg/hr through the feed throat. The TiO₂ was fed to the extruder at a rate of 1.2 kg/hr through a side arm at the 5^{th} barrel. The extruder temperature was set at 100 °C and the screw speed was set at 300 rpm. The extrudate was cut into master batch pellets using a strand pelletizer and collected.

### Comparative Examples C1-C5 and Examples 1-2: Aqueous opacifier dispersions

Aqueous opacifier dispersions were prepared in each of **Comparative Examples C1-C5** and **Examples 1-2** using a Coperion ZSK26 twin screw extruder. The polycaprolactone or polycaprolactone/TiO₂ composite master pellets, as noted in **TABLE 1,** were fed into the extruder at a rate of 4.2 kg/hr through the feed throat, together with the processing surfactant at the rate noted in **TABLE 1.** Deionized water was injected into the extruder at a rate of 0.42 kg/hr at the 4^{th} barrel. A second deionized water stream was injected into the extruder at a rate of 4.2 kg/hr at the 9^{th} barrel. The extruder temperature was set at 100 °C and the screw speed was set at 400 rpm. The product aqueous opacifier dispersion was collected at the outlet of the extruder and used as collected. The opacifier particles in **Comparative Examples C2 and C3** failed to disperse in the aqueous medium.

The Dv50 particle size of the dispersed opacifier particles in **Comparative Examples C1, C4-C5 and Examples 1-2** was measured by dynamic light scattering using a Beckman LS230 particle size analyzer. The results are provided in **TABLE 1.**

**TABLE 1**

| **Ex.** | **Ingredients (in kg/hr)** | | | | | | | | | **Dv50 (nm)** | **Solids (wt%)** |
|---|---|---|---|---|---|---|---|---|---|---|---|
| | **Polymer** | | | **Water** | | **Surfactant** | | | | | |
| | **A** | **B** | **C** | **Initial** | **Dilution** | **D** | **E** | **F** | **G** | | |
| **C1** | 4.2 | - | - | 0.42 | 4.2 | 0.084 | - | - | - | 338 | 47.2 |
| **C2** | 4.2 | - | - | 0.42 | 4.2 | - | 0.420 | - | -- | - | 45.5 |
| **C3** | 4.2 | - | - | 0.42 | 4.2 | - | - | 0.294 | - | - | 16.1 |
| **C4** | - | 4.2 | - | 0.42 | 4.2 | - | - | - | 0.294 | 412 | 46.1 |
| **C5** | - | 4.2 | - | 0.42 | 4.2 | - | - | - | 0.420 | 360 | 45.5 |
| **1** | - | - | 4.2 | 0.42 | 4.2 | - | - | - | 0.294 | 1352 | 46.1 |
| **2** | - | - | 4.2 | 0.42 | 4.2 | -- | - | - | 0.420 | 1080 | 45.5 |
| ^{A} CAPA^{™} 6800 | | | | | | | | | | | |
| ^{B} CAPA^{™} 6500 | | | | | | | | | | | |
| ^{C} CAPA^{™} 6500/TiO₂ composite from **Synthesis S1** | | | | | | | | | | | |
| ^{D} PVA (GH-17S) | | | | | | | | | | | |
| ^{E} ECOSURF^{™} EH-40 | | | | | | | | | | | |
| ^{F} DOWFAX^{™} AS-801 | | | | | | | | | | | |
| ^{G} Pluronic F-108 | | | | | | | | | | | |

### Comparative Examples CF1-CF5 and Examples F1-F2: Shampoo formulations

Shampoo formulations were prepared in each of **Comparative Examples CF1-CF5** and **Examples F1-F2** by combining the components in the amounts listed in **TABLE 2.**

**TABLE 2**

| **Ex.** | **Component** | | | | | | | |
|---|---|---|---|---|---|---|---|---|
| | **NaCl (wt%)** | **SLES (wt%)** | **CAPB (wt%)** | **Gycerine (wt%)** | **Euxyl^{®} PE 9010 (wt%)** | **Opacifier** | | **DI Water (wt%)** |
| | | | | | | **Type** | **(wt%)** | |
| **CF1** | 3.0 | 15.0 | 2.5 | 0.5 | 0.5 | **-** | 0 | q.s. 100 |
| **CF2** | 3.0 | 15.0 | 2.5 | 0.5 | 0.5 | **Commercial¹** | 10.0 | q.s. 100 |
| **CF3** | 3.0 | 15.0 | 2.5 | 0.5 | 0.5 | **Example C1** | 10.0 | q.s. 100 |
| **CF4** | 3.0 | 15.0 | 2.5 | 0.5 | 0.5 | **Example C4** | 10.0 | q.s. 100 |
| **CF5** | 3.0 | 15.0 | 2.5 | 0.5 | 0.5 | **Example C5** | 10.0 | q.s. 100 |
| **F1** | 3.0 | 15.0 | 2.5 | 0.5 | 0.5 | **Example 1** | 10.0 | q.s. 100 |
| **F2** | 3.0 | 15.0 | 2.5 | 0.5 | 0.5 | **Example 2** | 10.0 | q.s. 100 |
| ¹ OPULYN^{™} 301 opacifier available from The Dow Chemical Company | | | | | | | | |

### Performance Testing

The opacity of the shampoo formulations from each of **Comparative Examples CF1-CF5** and **Examples F1-F2** was analyzed by adding a 3 mL sample of each shampoo formulation to a separate 5 mL glass bottle. The bottles were then placed on a fixed holder in front of a camera. The opacity of the shampoo formulations was tested using PICA II (Phase Identification and Characterization Apparatus). The photos were taken under consistent light condition with front photographing. The gray value was calibrated from 0 to 255 where higher value corresponds to higher opacity. The gray value for each sample is reported in **TABLE 3.**

**TABLE 3**

| **Example** | **Gray value** |
|---|---|
| **CF1** | 43 |
| **CF2** | 215 |
| **CF3** | 167 |
| **CF4** | 185 |
| **CF5** | 187 |
| **F1** | 217 |
| **F2** | 221 |

## Claims

1. A composite opacifier dispersion, comprising:
a dispersion medium;
a processing surfactant; and
a plurality of composite opacifier particles, wherein the composite opacifier particles comprise metal oxide particles that are partially or completely encapsulated by a polycaprolactone polymer; wherein the metal oxide particles are selected from the group consisting of zinc oxide, titanium oxide and mixtures thereof; wherein the metal oxide particles have a z average particle size of > 100 nm as measured by dynamic light scattering; and wherein the composite opacifier particles have a z average particle size of > 150 nm to 2,500 nm as measured by dynamic light scattering.

2. An aqueous personal care rinse off composition, comprising:
a dermatologically acceptable aqueous vehicle;
a dermatologically acceptable cleaning surfactant; and
the composite opacifier dispersion according to claim 1.

3. The aqueous personal care rinse off composition of claim 2, wherein the aqueous personal care rinse off composition is selected from the group consisting of a shampoo, a conditioning shampoo, a body wash formulation, an exfoliating body wash formulation, a facial wash formulation, an exfoliating facial wash formulation, a liquid hand soap formulation, a sulfate-free cleansing formulation and a mild cleansing formulation.

4. The aqueous personal care rinse off composition of claim 3, wherein the dermatologically acceptable cleaning surfactant is selected from the group consisting of alkyl polyglucosides, glycinates, betaines, taurates, glutamates, sarcosinates, isethionates, sulfoacetates, alaninates, amphoacetates, sulfates, sulfonates, succinates, fatty alkanolamides and mixtures thereof.

5. The aqueous personal care rinse off composition of claim 4, wherein the metal oxide particles are titanium dioxide particles.

6. The aqueous personal care rinse off composition of claim 5, further comprising a rheology modifier.

7. The aqueous personal care rinse off composition of claim 6, wherein the polycaprolactone polymer has a weight average molecular weight of 10,000 to 1,000,000 Daltons.

8. The aqueous personal care rinse off composition of claim 6, wherein the polycaprolactone polymer has a weight average molecular weight of 20,000 to 200,000 Daltons.

9. The aqueous personal care rinse off composition of claim 6, wherein the polycaprolactone polymer has a weight average molecular weight of 25,000 to 150,000 Daltons.

10. A method of cleaning at least one of mammalian skin and hair, comprising:
(a) applying an aqueous personal care rinse off composition according to claim 2 to the skin or hair of a mammal; and
(b) rinsing the aqueous personal care rinse off composition from the skin or hair with a rinse water.

## Patentansprüche

1. Zusammengesetzte Trübungsmitteldispersion, umfassend:
ein Dispersionsmedium;
ein Verarbeitungstensid; und
eine Vielzahl von zusammengesetzten Trübungsmittelpartikeln, wobei die zusammengesetzten Trübungsmittelpartikel Metalloxidpartikel umfassen, die teilweise oder vollständig von einem Polycaprolactonpolymer eingekapselt sind; wobei die Metalloxidpartikel aus der Gruppe ausgewählt sind, bestehend aus Zinkoxid, Titanoxid und Mischungen davon; wobei die Metalloxidpartikel eine z-durchschnittliche Partikelgröße von > 100 nm, wie durch dynamische Lichtstreuung gemessen, aufweisen; und wobei die zusammengesetzten Trübungsmittelpartikel eine z-durchschnittliche Partikelgröße von > 150 nm bis 2.500 nm, wie durch dynamische Lichtstreuung gemessen, aufweisen.

2. Wässrige Körperpflegeabspülzusammensetzung, umfassend:
ein dermatologisch verträgliches wässriges Vehikel;
ein dermatologisch verträgliches Reinigungstensid; und
die zusammengesetzte Trübungsmitteldispersion nach Anspruch 1.

3. Wässrige Körperpflegeabspülzusammensetzung nach Anspruch 2, wobei die wässrige Körperpflegeabspülzusammensetzung aus der Gruppe ausgewählt ist, bestehend aus einem Shampoo, einem Pflegeshampoo, einer Körperwaschformulierung, einer Peeling-Körperwaschformulierung, einer Gesichtswaschformulierung, einer Peeling-Gesichtswaschformulierung, einer flüssigen Handseifenformulierung, einer sulfatfreien Reinigungsformulierung und einer milden Reinigungsformulierung.

4. Wässrige Körperpflegeabspülzusammensetzung nach Anspruch 3, wobei das dermatologisch verträgliche Reinigungstensid aus der Gruppe ausgewählt ist, bestehend aus Alkylpolyglucosiden, Glycinaten, Betainen, Tauraten, Glutamaten, Sarcosinaten, Isethionaten, Sulfoacetaten, Alaninaten, Amphoacetaten, Sulfaten, Sulfonaten, Succinaten, Fettalkanolamiden und Mischungen davon.

5. Wässrige Körperpflegeabspülzusammensetzung nach Anspruch 4, wobei die Metalloxidpartikel Titandioxidpartikel sind.

6. Wässrige Körperpflegeabspülzusammensetzung nach Anspruch 5, ferner umfassend einen Rheologiemodifikator.

7. Wässrige Körperpflegeabspülzusammensetzung nach Anspruch 6, wobei das Polycaprolactonpolymer eine gewichtsmittlere Molekularmasse von 10.000 bis 1.000.000 Dalton aufweist.

8. Wässrige Körperpflegeabspülzusammensetzung nach Anspruch 6, wobei das Polycaprolactonpolymer eine gewichtsmittlere Molekularmasse von 20.000 bis 200.000 Dalton aufweist.

9. Wässrige Körperpflegeabspülzusammensetzung nach Anspruch 6, wobei das Polycaprolactonpolymer eine gewichtsmittlere Molekularmasse von 25.000 bis 150.000 Dalton aufweist.

10. Verfahren zum Reinigen von mindestens einem von Haut und Haar von Säugetieren, umfassend:
(a) Auftragen einer wässrigen Körperpflegeabspülzusammensetzung nach Anspruch 2 auf die Haut oder das Haar eines Säugetiers; und
(b) Abspülen der wässrigen Körperpflegeabspülzusammensetzung von der Haut oder dem Haar mit einem Spülwasser.

## Revendications

1. Dispersion opacifiante composite, comprenant :
un milieu de dispersion ;
un agent tensioactif de traitement ; et
une pluralité de particules opacifiantes composites, dans laquelle les particules opacifiantes composites comprennent des particules d'oxyde métallique qui sont partiellement ou totalement encapsulées par un polymère de polycaprolactone ; dans laquelle les particules d'oxyde métallique sont choisies dans le groupe constitué d'oxyde de zinc, oxyde de titane et mélanges de ceux-ci ; dans laquelle les particules d'oxyde métallique ont une taille de particules moyenne en z de > 100 nm, telle que mesurée par diffusion dynamique de la lumière ; et dans laquelle les particules opacifiantes composites ont une taille de particules moyenne en z de > 150 nm à 2 500 nm, telles que mesurée par diffusion dynamique de la lumière.

2. Composition aqueuse de soins personnels à rincer, comprenant :
un véhicule aqueux dermatologiquement acceptable ;
un agent tensioactif de nettoyage dermatologiquement acceptable ; et
la dispersion opacifiante composite selon la revendication 1.

3. Composition aqueuse de soins personnels à rincer selon la revendication 2, dans laquelle la composition aqueuse de soins personnels à rincer est choisie dans le groupe constitué d'un shampooing, d'un shampooing revitalisant, d'une formulation de lavage du corps, d'une formulation exfoliante de lavage du corps, d'une formulation de lavage du visage, d'une formulation exfoliante de lavage du visage, d'une formulation de savon liquide pour les mains, d'une formulation de nettoyage sans sulfate et d'une formulation de nettoyage doux.

4. Composition aqueuse de soins personnels à rincer selon la revendication 3, dans laquelle l'agent tensioactif de nettoyage dermatologiquement acceptable est choisi dans le groupe constitué d'alkyl polyglucosides, glycinates, bétaïnes, taurates, glutamates, sarcosinates, iséthionates, sulfoacétates, alaninates, amphoacétates, sulfates, sulfonates, succinates, alcanolamides gras et mélanges de ceux-ci.

5. Composition aqueuse de soins personnels à rincer selon la revendication 4, dans laquelle les particules d'oxyde métallique sont des particules de dioxyde de titane.

6. Composition aqueuse de soins personnels à rincer selon la revendication 5, comprenant en outre un modificateur de rhéologie.

7. Composition aqueuse de soins personnels à rincer selon la revendication 6, dans laquelle le polymère de caprolactone a une masse moléculaire moyenne en poids de 10 000 à 1 000 000 Daltons.

8. Composition aqueuse de soins personnels à rincer selon la revendication 6, dans laquelle le polymère de caprolactone a une masse moléculaire moyenne en poids de 20 000 à 200 000 Daltons.

9. Composition aqueuse de soins personnels à rincer selon la revendication 6, dans laquelle le polymère de caprolactone a une masse moléculaire moyenne en poids de 25 000 à 150 000 Daltons.

10. Procédé de nettoyage d'au moins l'un parmi la peau et les cheveux de mammifère, comprenant :
(a) l'application d'une composition aqueuse de soins personnels à rincer selon la revendication 2 sur la peau ou les cheveux d'un mammifère ; et
(b) le rinçage de la composition aqueuse de soins personnels à rincer de la peau ou des cheveux avec une eau de rinçage.
